# EUROPEAN PATENT APPLICATION

(11) **EP 4 663 146 A1**
(43) Date of publication of application: **17.12.2025**
(21) Application number: 24182326.9
(22) Date of filing: 14.06.2024
(51) Int. Cl.: A61B 34/20, A61B 17/00

(54) **LENGTH-ADJUSTABLE SURGICAL POINTER**

(71) Applicant: Stryker European Operations Limited, Carrigtwohill, Co. Cork T45 HX08 (IE)
(72) Inventor: KHORWAL, Renu, 110063 New Delhi (IN); KUMAR, Pankaj, 11005 New Delhi (IN); BEDI, Inderjeet Singh, 110032 Delhi (IN); GUPTA, Rachana, 781024 Assam (IN); RAINA, Ravi Kiran, 201301 Noida (IN)
(74) Representative: Röthinger, Rainer

(57) **Abstract**

A length-adjustable surgical pointer 200 is provided. The pointer 200 comprises a shaft 250 carrying a pointing tip 252 and a handle 210 supporting the shaft 250. At least a portion of the shaft 250 is movable in a translatory manner relative to the handle 210 so as to adjust a distance between the pointing tip 252 and the handle 210.

## Description

### Technical Field

The present disclosure generally relates to pointers for navigated surgery. In particular, a surgical pointer, a system comprising the pointer, a method for determining a pointer length and a computer program product are presented.

### Background

In navigated surgery, surgical pointers are used to calibrate surgical instruments, to indicate anatomic points of a patient and for similar purposes. To this end, a pointer equipped with a tracker is moved to touch an object (e.g., an instrument, a calibration tool or an anatomic point) with a tip of the pointer. In this procedure, the pose of the pointer is tracked and based on a known geometric relationship between the tracker and the tip of the pointer, the position of the touched object can be determined (e.g., calibrated), for example in a tracking coordinate system used for surgical navigation.

A precise determination of the position of the touched object in the tracking coordinate system is of high importance to minimize a potential health risk in a subsequent navigation procedure. Therefore, the surgical results also depend on a precise knowledge of geometric relationship between the pointing tip and the tracker on the pointer.

An important parameter of a surgical pointer is the pointer length. From a subjective perspective of a user, the pointer length is defined as a distance between an pointer handle and the tip of the pointer, but the pointer length can also be indicated for example in terms of the length of a pointer shaft extending from the pointer handle.

An appropriate pointer length depends on the type of surgery to be performed and the subjective preferences of the person using the pointer. For example, in spinal surgery different lengths are required for marking anatomic points in the cervical region and in the lumbar region of the spine. Furthermore, an "ergonomic" length is a subjective aspect that strongly depends on a user's preferences. For these reasons, pointers a provided in sets that comprise pointers of different lengths.

Therefore, a plurality of different pointers must often be provided for a single surgical intervention, and despite the plurality of pointers, there is still a possibility that none of the available pointers has the desired dimension, e.g., an optimal length, for a particular user and a particular surgical application.

### Summary

There is a need for an improved surgical pointer addressing at least some of the aforementioned or other problems.

According to a first aspect, a length-adjustable surgical pointer is provided. The pointer comprises a shaft carrying a pointing tip and a handle supporting the shaft. At least a portion of the shaft is movable in a translatory manner relative to the handle so as to adjust a distance between the pointing tip and the handle.

The pointer length depends on the currently adjusted distance between the pointing tip and the handle of the pointer. The pointer length can be defined in different ways, for example based on a distance between the pointer handle and the pointing tip, in terms of the length of the pointer shaft, based on a vector extending between the pointing tip and a tracker on the handle, and so on.

In some variants, the pointer may comprise a user-operable input element (e.g., a button) configured to signal to a computing device (e.g., of a tracking system) when a position of the pointing tip is to be acquired. To this end, the pointer may comprise circuitry configured for wired or wireless communication. In some implementations, this communication is effected via an active tracker carried by the pointer, that is electrically coupled to the input element.

At least the portion of the shaft capable of the translatory movement may be manually or automatically movable relative to the handle, e.g., by hand or by an electric actuator. In some variants, the shaft may be at least partially movable into or out of the handle for adjusting the distance between the pointing tip and the handle. For example, the shaft may have a predefined length (e.g., the shaft may be a rigid rod of a predefined length), and at least a portion of the predefined length may be movable into or out of the handle.

The pointer handle may comprise a handle grip configured to be held by a user and a handle body (e.g., in a pistol-type configuration). In such a configuration, the handle body may be configured to at least partially receive the shaft. In some examples, the body may comprise a tube configured to at least partially receive the shaft in a telescoping manner. In some variants, at least one of the tube and the grip may be detachably attached to the body. In some variants, at least the grip and the body may be formed as a single piece.

Alternatively or additionally, the shaft may be at least partially configured to perform a telescopic movement for adjusting the distance between the pointing tip and the handle, for example, like a telescopic antenna known from cars or radio sets. In some variants, the shaft may be at least partially movable into the handle via translatory movement, or may be capable of a telescopic movement, or a combination thereof.

In some variants, the shaft may comprise multiple sections, e.g., two, three, four, five or more sections. Each section may have a respective predefined length to enable a discrete adjustment of the distance between the pointing tip and the handle (i.e., to adjust the length of the pointer), based on the respective predefined lengths. The respective predefined lengths may be the same, allowing a fast and easy determination of the length adjustment. Alternatively, the respective predefined lengths may vary to combine fast and detailed length-adjustment of the pointer. In one example, the respective predefined lengths may be larger the closer they are to the pointing tip. In another example, the respective predefined lengths may be smaller the closer they are to the pointing tip.

At least some of the sections may be telescopic sections, i.e., at least a portion of a telescopic section may be movable into and out of another telescopic section. The telescopic sections may be individually actuatable, e.g., in sequence from the innermost to the outermost of the telescopic sections.

Additionally or alternatively, the shaft may be movable in a continuous translatory movement to enable a continuous adjustment of the distance between the pointing tip and the handle, i.e., the length of the pointer.

In some variants, the shaft may comprise at least one optical indicator configured to optically indicate information associated with a currently adjusted length of the pointer. For example, the at least one optical indicator may indicate the distance between the pointing tip and the handle (e.g., a predefined point on the handle), an effective (e.g., free) shaft length or a vector stretching between a tracker mounted on the pointer and the pointing tip. Based on the indicated information and at least one of a known geometry of the handle, e.g., via manufacturing data or a model of the pointer, and a reference distance, e.g., a distance indicated in previously generated image data and associated with a known previous length of the pointer, the currently adjusted length of the pointer may be determined.

In some variants, the at least one optical indicator may comprise at least one of a length scale, a color coding, and a coding by an optical pattern. The at least one optical indicator may circumferentially extend about a surface of the shaft so that the at least one optical indicator can be seen from every viewing direction. For example, the at least one optical indicator may comprise a length scale comprising multiple length indications spaced apart from each other along a length of the shaft, wherein each indication may extend circumferentially about a surface of the shaft, e.g., like a cylindrical measuring cup with the indications extending all around the measuring cup. In the case of a length scale, the distance between the pointing tip and the handle may be directly determined by reading out the visible length scale.

Additionally or alternatively, the shaft may comprise multiple sections discernable by optical indicators, e.g., by at least one of a color coding and a coding by optical patterns. For example, neighboring sections may comprise at least one of different colors (e.g., red, blue, green, black, white) and different optical patterns (e.g., patterns comprising different geometrical structures like triangles, squares or other geometric structures), which may be discernable by a camera. In some variants, at least one of the different colors and different optical patterns may be arranged in a predefined sequence. For example, in case of a shaft comprising three or more sections, neighboring sections may have at least one of alternating colors, e.g., alternating between black and white, and alternating optical patterns, e.g., alternating between a pattern of squares and a pattern of triangles. In some variants, each section may have at least one of a unique color and a unique optical pattern.

In some variants, at least some of the colors and the optical patterns may be associated with a predetermined pointer length, so that each section of the shaft comprises at least one of a color and a pattern with an associated length. For example, the shaft may comprise three sections, each section having a different color as respective optical indicator (e.g., red, black and white). The red section may have a length of 4 cm, the black section may have a length of 2 cm and the white section may have a length of 1 cm. The red section may be the most proximal distance, the black section the middle section and the white section the most distal section. The white section may comprise the pointing tip. Based on an identification of the visible colors, the total distance between the pointing tip and the handle may easily be determined, e.g., 1 cm, 3 cm or 7 cm. In such or other variants, a vector, a model or other information indicating the pointer length may then be determined, selected or adjusted based on the determined currently adjusted distance between the pointing tip and the handle.

In some variants, the respective optical indicator(s) may be associated with the section length(s) (e.g., 1 cm, 2cm and 4 cm according to the previous example) of the respective section, and the total distance between the pointing tip and the handle may be determined by a computing device. Alternatively, to reduce computation load on the computing device, the respective optical indicator(s) may be associated with the respective total distance(s), e.g., 1 cm, 3 cm and 7 cm, according to the previous example. In this variant, the pointer length may be determined based on the largest of the lengths associated with the visible optical indicator(s).

In some variants, the shaft may have more than three sections, e.g., 4, 5, 6 or more sections. The length of a single section may be in the range of 5 mm to 10 cm (e.g., between 1 cm and 6 cm).

In some variants, the length-adjustable pointer may further comprise a locking element configured to releasably lock the shaft relative to the handle against an unintentional translatory movement therebetween. The locking may be such that a certain minimum amount of force is required for a length adjustment in the locked state, or such that the locked state prevents any length adjustment without potentially causing damage to the pointer.

The locking element may be a mechanical locking element, such as a locking screw. In some variants, one of the shaft and the locking element may comprise a preloaded snap-in element and the other one comprises a corresponding receiving element configured to releasably receive the snap-in element. For example, the shaft may comprise at least one depression forming the receiving element and the locking element may comprise the preloaded snap-in element, wherein the snap-in element is configured to engage the at least one depression. In another example, the shaft may comprise multiple snap-in elements or depressions spaced apart along a length of the shaft. The handle, for example a tube of the handle configured to receive the shaft, may comprise at least one corresponding element.

The locking element may be manually actuatable to at least one of lock or unlock the shaft relative to the handle. For example, the locking element may be movable against a preloaded spring. The locking element may comprise a button. In other variants, the locking element comprises a screw.

The pointer may further comprise one or more mechanical stopper elements. For example, the pointer may comprise a distal mechanical stopper defining a most distal position of the pointing tip relative to the handle, i.e., preventing further translatory movement of the shaft relative to the handle in a distal direction when the pointing tip is located at the most distal position relative to the handle. The pointer may additionally or alternatively comprise a proximal mechanical stopper defining a most proximal position of the pointing tip relative to the handle, i.e., preventing further translatory movement of the shaft relative to the handle in a proximal direction, when the pointing tip is located at the most proximal position.

In some variants, the length-adjustable pointer may further comprise a tracker configured to be detected by a surgical tracking system, or an interface for receiving the tracker. The tracker may be fixedly integrated in the pointer or may be detachable therefrom. The tracker may be an optical tracker or an electromagnetic tracker. The tracker may be located at a predetermined position of the pointer.

A vector, also referred to as tracker vector herein, between the tracker and the pointing tip may indicate the (currently adjusted) length of the pointer (e.g., in terms of the vector length). The vector may be indicated in a coordinate system tied to the pointer (e.g., in a tracker coordinate system or a pointer coordinate system). The tracker vector may be determined based on the information indicated by the at least one optical indicator.

The tracker vector may be defined based on a variable vector and a constant vector (e.g., as a sum, difference or product). The variable vector may extend between the pointing tip and a reference point of the handle, and thus may vary dependent on the relative translatory movement between the pointing tip and the handle. The variable vector may be determined based on the information indicated by the at least one optical indicator. The constant vector may extend between the reference point of the handle and a reference point of the tracker (e.g., an origin of the tracker coordinate system). The constant vector may be determined from manufacturing data or a model of the pointer.

According to further aspect, a system is provided. The system comprises the length-adjustable pointer comprising at least the at least one optical indicator as described herein. The system further comprises a camera configured to generate image data indicative of the at least one optical indicator.

The system may further comprise a computing device configured to receive the image data and determine information associated with a currently adjusted length of the pointer based at least in part on the at least one optical indicator (as described herein) in the image data.

In some variants, the pointer of the system may further comprise a tracker or may have received a tracker via the interface as described herein. The computing device may be configured to determine a relative position between the tracker and the pointing tip, i.e., the tracker vector, based at least in part on the received image data. For example, the computing device may be configured to determine the relative position between the pointing tip and the tracker based at least in part on the received image data and at least one of manufacturing data and a model of the pointer.

Additionally or alternatively, the computing device may be configured to receive calibration image data, e.g., image data indicative of a pose of the tracker and a pose of a trackable calibration tool when touching the calibration tool at a predetermined position with the pointing tip. The computing device may be configured to determine the tracker vector based at least in part on the calibration image data.

In some variants, the computing device may be configured to determine the information associated with the currently adjusted length of the pointer by detecting (e.g., identifying) the at least one optical indicator in the image data and by consulting a mapping between different optical indicators and different items of information associated with adjusted pointer lengths. In some implementations, each optical indicator is uniquely mapped onto one such item of information. The different items of information may comprise at least one of different pointer models, different tracker vectors, different numerical length values, and so on.

In some variants, the computing device may be configured to detect a change of the currently adjusted pointer length relative to a previously adjusted pointer length based on a comparison between a currently visible at least one optical indicator and a previously visible at least one optical indicator. In some implementations, the detection triggers at least one of a request to the user for a re-calibration of the pointer (e.g., using a calibration tool) and a notification of a navigation algorithm.

In some variants, the computing device may be configured to automatically determine information associated with a length of the pointer in reaction to receiving image data. The computing device may further be configured to automatically compare the determined information with previously determined information and, in case a change is detected, e.g., to trigger an adaption of at least one of model of the pointer and the tracker vector as described herein.

In some variants, the computing device may automatically determine information associated with a currently adjusted length of the pointer in reaction to detecting a change in the at least one optical indicator visible in the image data. The change may be detected based on one of a change of the color or pattern of a visible shaft section closest to the handle, a changed sequence of colors or patterns along the visible shaft sections, or a changed number of visible shaft sections. In case a change is detected, the computing device may automatically determine further information associated with the currently adjusted pointer length as described herein, e.g., at least one of the currently adjusted distance between the pointing tip and the handle and the adjusted tracker vector and, optionally, automatically trigger an adaption of at least one of model and the tracker vector as described herein.

Additionally or alternatively, the computing device may determine information associated with a currently adjusted pointer length in reaction to a manual input from a user, e.g., from a surgeon.

In some variants, the computing device may be configured to automatically initiate a validation workflow to validate information associated with a currently assumed or determined pointer length. The validation workflow may comprise generating new image data indicative of the at least one optical indicator, referred to as validation image data herein, and determining information associated with the currently assumed or determined pointer length based on the validation image data. The computing device may be configured to automatically trigger a validation workflow (e.g., upon start-up of a navigation workflow). The computing device may alternatively or additionally be configured to trigger the validation workflow at predetermined time intervals, e.g., by associating received image data with a time stamp and triggering the validation workflow when the age of the youngest time stamp is above a threshold.

The validation workflow may comprise validation of any information associated with a currently assumed or determined pointer length (e.g., a validation of at least one of the distance between the pointing tip and the handle, the model of the pointer and the tracker vector as described herein). Such validation workflows reduce the probability of working with an actual pointer length that deviates from an assumed pointer length used by a navigation workflow.

In some variants, when the pointer comprises the tracker or has received the tracker via the interface, the computing device may be configured to receive data indicative of a pose of the tracker and to generate navigation instructions based at least in part on the indicated pose and the information associated with the currently adjusted length of the pointer. In some variants, generating navigation instructions may comprise adjusting a model of the pointer based on the information associated with the currently adjusted length of the pointer, and generating navigation instructions based on the adjusted model and the indicated pose of the tracker. The data indicative of the pose of the tracker may be tracking data based on optical or electromagnetic tracking.

According to another aspect, a computer-implemented method for determining information associated with a currently adjusted length of the length-adjustable pointer as described herein is provided. The method comprises receiving image data indicative of the at least one optical indicator. The method further comprises determining information associates with the currently adjusted length of the pointer based at least in part on the at least one optical indicator in the received image data.

In some variants, the length-adjustable pointer may comprise the tracker or a tracker received vis the interface as described herein. In such variants, the method may further comprise receiving data indicative of a pose of the tracker and determining navigation instructions based at least in part on the indicated pose and the information associated with the currently adjusted length of the pointer.

The determination of the distance and the generation of the navigation instructions may comprise any steps or techniques described herein with reference to the computing device of the system described above.

According to another aspect, a computer program product is provided. The computer program product comprises instructions which, when the program is executed by a processor, cause the processor to carry out the method as described herein.

### Brief Description of the Drawings

Further details, advantages and aspects of the present disclosure will become apparent from the following embodiments taken in conjunction with the drawings, wherein:
- Fig. 1: shows a system comprising a length-adjustable surgical pointer, a camera and a computing device;
- Fig. 2: shows the length-adjustable pointer of Fig 1 in more detail;
- Fig. 3A: shows a partially exploded view of the length-adjustable pointer of Figs. 1 and 2;
- Fig. 3B: shows the shaft of the length adjustable pointer of Figs 1 to 3A comprising multiple optical indicators in the form of optical patterns;
- Fig. 4A: shows a flow diagram of a method for determining information associated with a currently adjusted length of the length-adjustable pointer;
- Figs. 4B-4D: show the length adjustable pointer of Figs. 1 to 3A, wherein each figure shows the pointer adjusted to a different length;
- Figs. 5A-5D: show a further length-adjustable surgical pointer, wherein each figure shows the pointer adjusted to a different length;
- Figs. 6A-6C: show a part of a further length-adjustable surgical pointer, wherein each figure shows the pointer adjusted to a different length;
- Fig. 7A: shows a detailed view of a portion of the pointer of Figs. 6A to 6C comprising a locking element;
- Fig. 7B: shows an exploded view of Fig. 7A; and
- Fig. 7C: shows a part of the locking element in more detail.

### Detailed Description

In the following description of exemplary embodiments, the same reference numerals are used to denote the same or similar components.

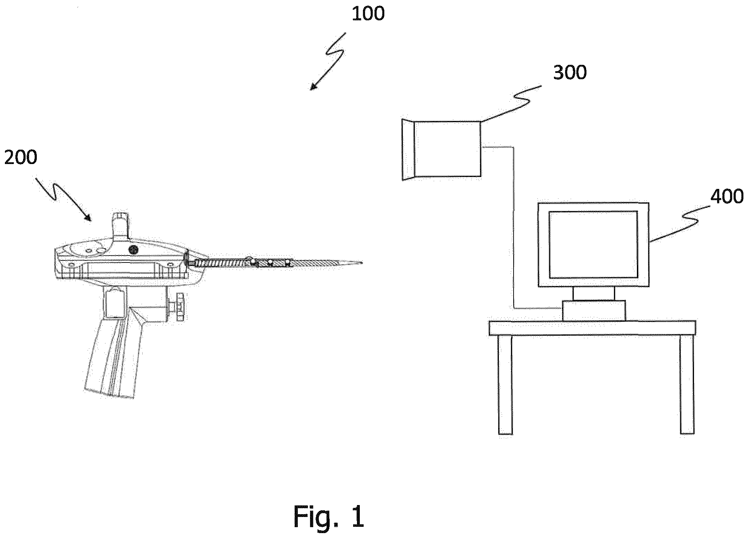

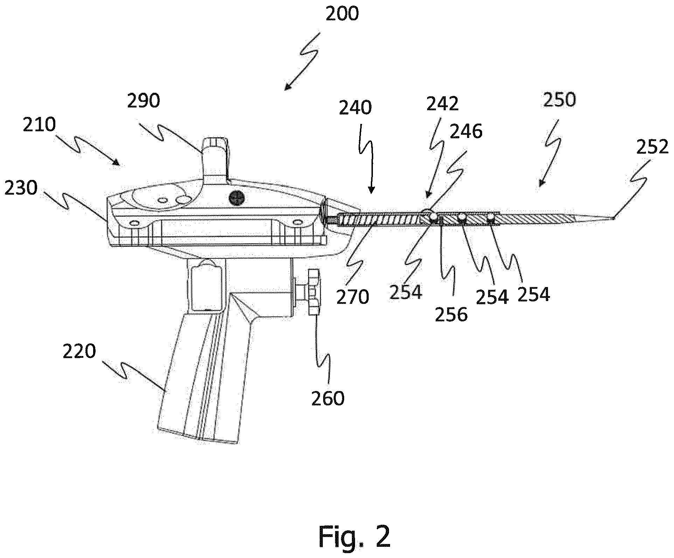

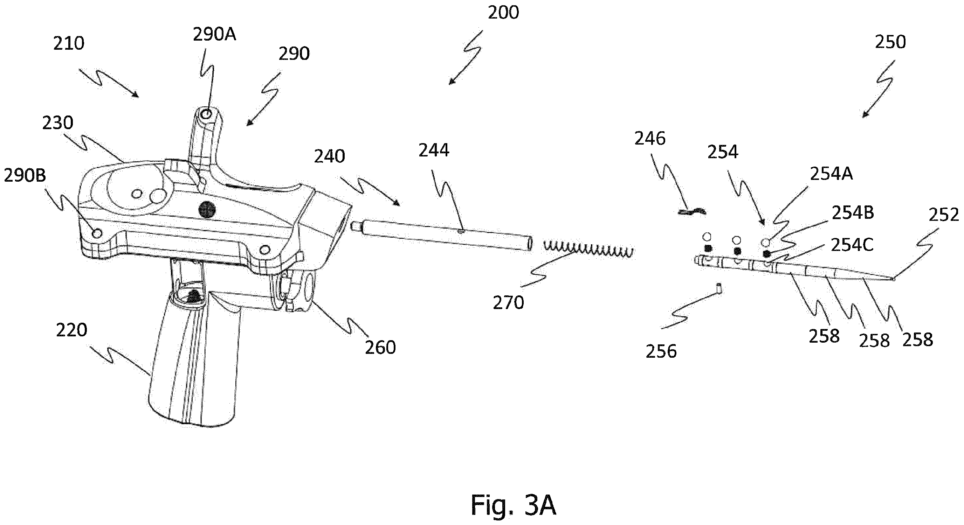

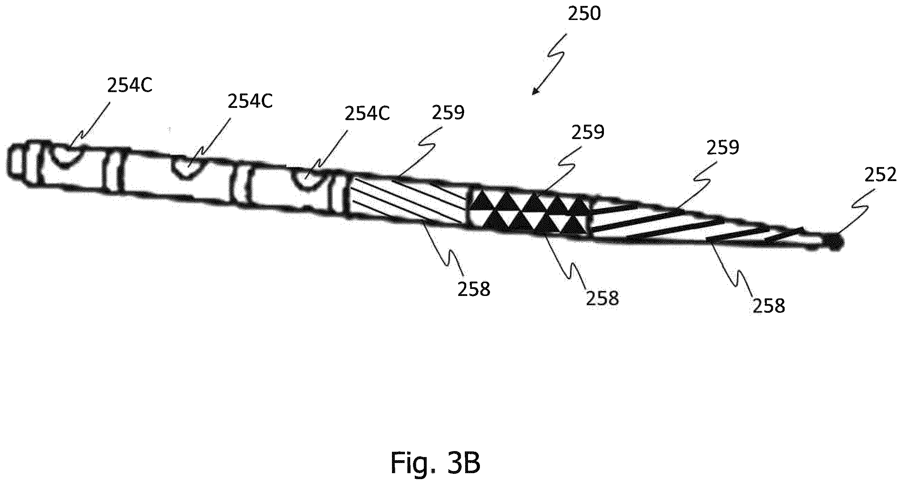

Fig. 1 shows a system 100 comprising a length-adjustable surgical pointer 200 and a camera 300 configured to take image data of the pointer 200. The system 100 further comprises a computing device 400 communicatively coupled to the camera 300. The pointer 200 of Fig. 1 is also shown in Fig. 2 in more detail and in Fig. 3A in an exploded view. Fig. 3B shows an aspect of the pointer 200 in more detail.

With reference to Figs. 2 and 3A, the pointer 200 comprises a pointer handle 210 and a pointer shaft 250. The handle 210 is formed from a handle grip 220, a handle body 230 and a handle tube 240. The handle body 230 carries a pre-loaded finger-operable button 260 for communication with the computing device 400.

The handle tube 240 is mounted to the handle body 230 and configured to slidably receive the shaft 250. The shaft 250 is a rigid, rod-like structure with a predefined length and comprises a pointing tip 252 at its distal end. In some variants, operation of the button 260 signals to the computing device 400 that the current position of the pointing tip 252 is to be acquired. To this end, the pointer 200 comprises circuitry configured for wired or wireless communication with the computing system 400 (e.g., via the camera 300).

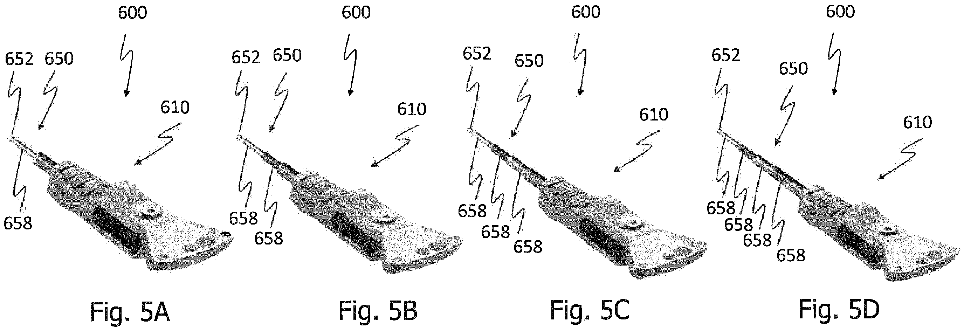

The shaft 250 further comprises multiple sections 258 dividing the shaft 250 along its axial extension between the pointing tip 252 and its opposite end. The shaft 250 further comprises multiple optical indicators 259 as shown in Fig. 3B (see also Figs. 5A to 5D for further examples of optical indicators). The optical indicators 259 shown in Fig 3B are realized as optical patterns detectable by the camera 300. As illustrated in Fig. 3B, each section 258 comprises a different optical indicator 259. Of course, instead of different optical patterns different colors could also be used. The lengths of the sections 258 (and, thus, of the optical indicators) can be identical or different and may match the distances between the locations of the snap-in elements 254 (i.e., of the bores 254C). These lengths and distances may be selected such that as the receiving element 244 engages a different snap-in element 254, a further optical indicator 259 becomes visible (out of the tube 240) or a previously visible optical indicator 259 disappears entirely (into the tube 240).

Each of the optical indicators 259 is associated with a predetermined pointer length. Based on the one or more optical indicators 259 actually visible to the camera 300 (i.e., not covered by the tube 240) and their associated lengths (as known - at least implicitly - to the computing device 400), a length of a visible part of the shaft 250 and, thus, the pointer length may be determined by the computing device 400, as will be explained below in greater detail.

The shaft 250 further comprises multiple preloaded snap-in elements 254 that are spaced apart from each other along a portion of the shaft 250 in an axial direction (i.e., along the axial extension of the shaft). Each snap-in element 254 is associated with one of the visual indicators 258 (and, thus, with a corresponding length adjustment of the pointer 200).

As becomes apparent from Fig. 3A, each snap-in element 254 comprises a ball 254A and a spring 254B that are located in a bore 256C of the shaft 250. The springs 254B preload the balls 254A in a direction orthogonal to the axial shaft direction. The shaft 250 further comprises a mechanical stopper 256 configured to define a most distal position of the shaft 250 relative to the handle 210 (i.e., to prevent the shaft 250 from exiting the tube 240).

The snap-in elements 254 are configured to cooperate with a complementary locking element 242 or similar locking structure in or on the tube 240. In the variant shown in Figs. 2 and 3A, the locking element 242 on the tube 240 comprises a receiving element 244 in the form of a hole in the wall of the tube 240. The receiving element 240 is configured to releasably engage with (i.e., receive) the ball 254A of one of the snap-in elements 254 so as to lock the shaft 250 relative to the handle 230 against an unintentional translatory movement therebetween. In association with the receiving element 244, an actuation element 246 in the form of a depressible button is provided on an outer surface of the tune 240. Upon actuation, the actuation element 246 presses the ball 254A accommodated in the receiving element 244 out of engagement with the receiving element 244 so as to enable a translatory movement between the shaft 250 and the handle 230. In this manner, an effective length of the shaft 250 can be adjusted by bringing the receiving element 244 into engagement with the ball 254A of another one of the snap-in elements 254.

By way of the mechanism described above, the shaft 250 is movable translatorily in discrete steps relative to the handle 210, i.e., into and out of the tube 240. In the example shown in Figs. 1 to 3B, the shaft 250 is biased by a spring 270 accommodated in the tube 240 in a distal direction away from the handle 210 and out of the tube 240. On the other hand, the shaft 250 is movable against the spring-bias relative to the handle 210 in a proximal direction, i.e., towards the body 230 of the handle 210 and into the tube 240.

With reference to Fig. 3A, the pointer 200 further comprises a tracker 290. In the variant shown in Fig. 3A, the tracker 290 is an active optical tracker comprising multiple light emitting elements 290A distributed over the handle body 230 and detectable by the camera 300. The light emitting elements 290A may be configured to emit light in the infrared of the visible spectrum. In other variants, the tracker 290 may be a passive optical tracker. In still other variants, the tracker 290 may be an electromagnetic tracker with one or more tracking coils.

The camera 300 illustrated in Fig. 1 is configured to generate image data indicative of both the light emitting elements 290A and the optical indicators 259 of the pointer 200. In other variants, the camera 300 may comprise two or more camera units, including a first camera unit configured to detect the light emitting elements 290A (e.g., in an infrared spectrum) and a second camera unit configured to detect the optical indicators 259 (e.g., in the visible spectrum). The camera 300 (e.g., one or both of the camera units) may be configured as a stereo camera. In some implementations, the camera 300 (e.g., one or both of the camera units) is configures as a video camera capable of acquiring image data sequences.

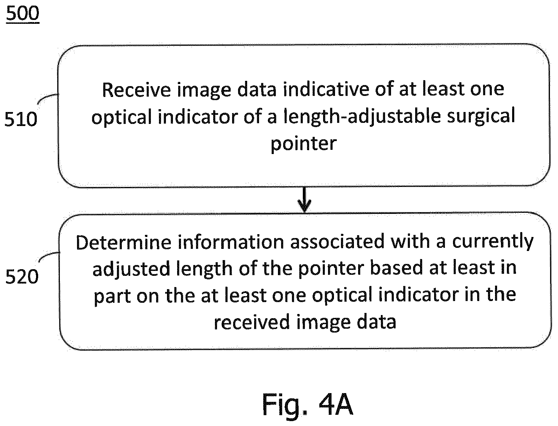

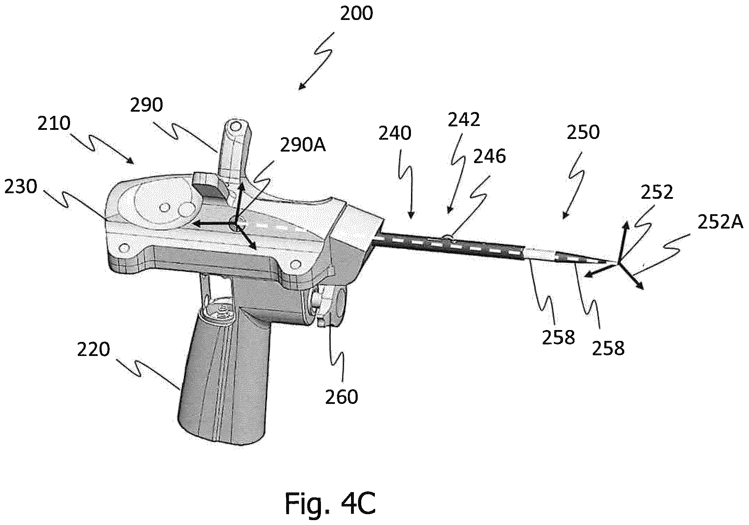

The computing device 400 is communicatively coupled to the camera 300 and configured to perform a method for determining information associated with a currently adjusted length of the pointer 200 as explained herein, in particular with reference to Fig. 4A.

Fig. 4A shows a flow diagram 500 of a method as performed by the computing device 400. The method may be performed under control of a computer program product stored on the computing device 400 and executed by a processor thereof (not shown in Fig. 1).

In a first step 510, the computing device 400 receives image data from the camera 300. The received image data is indicative of the at least one optical indicator 259 of the pointer 200. In some variants, the image data may additionally be indicative of the light emitting elements 290A of the tracker 290, or the image data indicative of the optical indicator 259 and of the light emitting elements 290A are taken by the camera 300 at different points in time (e.g., in different operational modes).

In a second step 520, the computing device 400 determines, based at least in part on the received image data, information associated with a currently adjusted length of the pointer 200. The determination in step 520 is based at least in part on the at least one optical indicator 259 indicated in the received image data.

With reference to the pointer 200 of Figs. 1 to 3B, the computing device 400 determines the information associated with a currently adjusted length of the pointer 200 by detecting (e.g., identifying) one or more optical indicators 259 that are visible in the image data and by consulting a mapping between different optical indicators 259 and different items of information associated with adjusted pointer lengths. The mapping may be pre-defined and stored by the computing device 400. In some implementations, each optical indicator 259 is uniquely mapped onto one such item of information. The different items of information may comprise different pointer models, different tracker vectors, different numerical length values, and so on. Each such item of information enables the computing device 400 to infer a currently adjusted pointer length and to use this pointer length for purposes such as calibration, tracking, navigation, and so on.

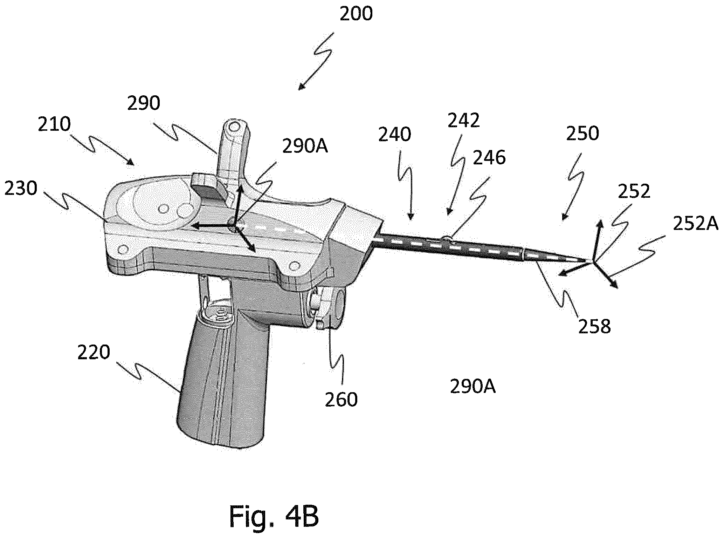

As an example, each tracker vector may stretch from the pointing tip 252 to the origin or another known point of a tracker coordinate system 290B defined by the tracker 290 (e.g., the coordinate system in which the coordinates of the light emitting elements 290A are defined), or vice versa. Three examples of tracker vectors extending between the pointing tip 252 and an origin of the tracker coordinate system 290B are shown as a white dashed lines in Figs. 4B to 4D. The pointing tip 252 is visualized to define the origin of a pointer coordinate system 252A. As can be determined in step 520 from the differently coded sections 258 visible in each case, the pointer 200 has been adjusted to a different pointer length in each of the Figs. 4B to 4D.

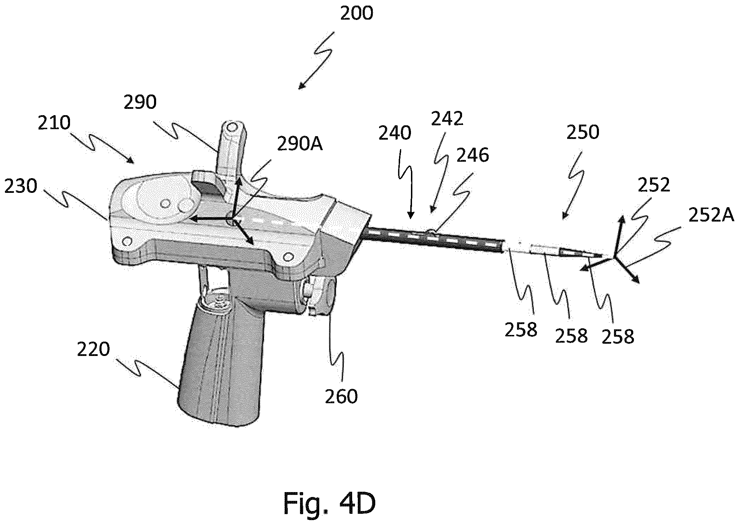

The different tracker vectors illustrated in Figs. 4B to 4D are associated with different snap-in elements 254 engaging the receiving element 244 and, thus, different pointer lengths. On the other hand, the different pointer lengths are also associated with the different optical indicators 259, as explained above. By detecting a certain optical indicator 259 in the image data, the associated tracker vector can thus be determined by the computing device 400 from the mapping stored by the computing device 400. If more than one optical indicator 259 is detected in the image data, only the tracker vector associated with the largest pointer length will be considered (see the examples of Fig. 3B and 4D, but other examples can be realized).

The tracker vectors (e.g., as defined dependent on the lengths of the sections 258) may be known from manufacturing data or from a model of the pointer 200. In some variants, the tracker vectors themselves may be considered as pointer models (e.g., as defining the possible pointer lengths) because in such variants, they embody all the information that needs to be known about the pointer 200 for determining the position of the pointing tip 252 in a tracking coordinate system: by vectorially adding the tracker vector determined in step 520 to the position of the tracker coordinate system 290B tracked in the tracking coordinate system, the position of the pointing tip 252 in the tracking coordinate system can be determined. In some variants, the tracking coordinate system can be defined to have its origin in a predefined geometric relationship relative to the camera 300.

In case of a pointer 200 with an optical indicator in the form of a length scale, the computing device 400 may determine the currently adjusted tracker length directly from the length scale as far as same is visible in the image data. As an example, the tracker length may be determined based on a length indication closest to the free end of the tube 240. The length indications closest to the free end of the tube 240 will change as the shaft 250 is moved into the tube 240 or out of the tube 240. The length thus determined may vectorially be added along a predefined direction to the tracked tracker coordinate system 290B to determine the position of the pointing tip 252 in the tracking coordinate system, in a similar manner as explained above.

The method 500 may comprise further, optional steps not shown in Fig. 4A. For example, the method 500 may comprise some or all of the steps executable by the computing device 400 described herein. In some variants, the computing device 400 is configured to receive data indicative of a pose of the tracker 290 (e.g., with reference to Figs. 4B to 4D, the pose of the tracker coordinate system 290B). The data may be received in the form of image data taken by the camera 300 and indicative of positions of the light emitting elements 290A that have a fixed and known position to the origin of the tracker coordinate system 290B. The computing device 400 may then determine the pose of the tracker 290 indicated in the data and generate calibration information, registration information, a visualization on a graphical user interface, navigation instructions, etc. based at least in part on the indicated pose and the information associated with the currently adjusted length of the pointer as determined in step 520 of Fig. 4A.

As an example, the user may adjust a certain pointer length as generally described above and then present the pointer 200 to the camera 300 in a pre-operative or calibration mode. The camera 300 takes image data of the pointer 200 and the computing device 400 performs the steps discussed above with reference to Fig. 4A to determine the currently adjusted tracker length (e.g., in terms of the tracker vector).

In a subsequent intra-operative mode, the user moves the pointing tip 252 of the tracked pointer 200 to an anatomic point of interest (e.g., of a spinal process) or one or more radio-opaque fiducials such as a bone screws. Once that point is reached, the user operates the button 260 to signal (e.g., via the light emitting elements 290A of the tracker 290 and, thus, via the camera 300) to the computing device 400 that the location of the anatomic point of interest or the fiducial is now to be acquired. The computing device 400 then takes a snapshot of the position of the tracker 290 as defined by the position and possibly orientation of the tracker coordinate system 290B in the tracking coordinate system. By subsequently adding the tracker vector determined earlier to that position of the tracker 290 in the tracking coordinate system (or using another item of information determined as indicated in step 520 of Fig. 4A), the exact position of the pointing tip 252 and, thus, of the anatomic point or the fiducial in the tracking coordinate system can be inferred, regardless of the currently adjusted pointer length. When there is a good line of sight to the optical indicators 259, the pointer length (i.e., the tracker vector) can be determined inter-operatively (e.g., from the image data acquired by the camera 300 when the user operates the button 260). The position of the anatomic point in the tracking coordinate system can then be used for generating navigation instructions (e.g., to guide a tracked surgical instrument, such as a drill, to the anatomic point of interest). In other examples, the position of the fiducial in the tracking coordinate system is used for registering within the tracking coordinate system computer tomography (CT) image data also indicative of the fiducial.

The length of the pointer 200 can be flexibly adjusted even during an ongoing surgical procedure if the need arises. To this end, the user simply repeats the pre-operative or calibration mode described above after a manual length adjustment and then continues with the intra-operative mode.

The computing device 400 may be configured to automatically initiate a validation workflow to validate information associated with a currently assumed or determined pointer length (e.g., to trigger a warning in case the pointer length is adjusted intraoperatively without repeating the pre-operative or calibration mode). The validation workflow comprises generating new image data by the camera 300 indicative of the at least one optical indicator 259, referred to as validation image data herein, and determining information associated with the currently assumed or determined pointer length based on the validation image data (e.g., as discussed with reference to Fig. 4A above). This information may then be compared to the corresponding information determined in the pre-operative or calibration mode. The computing device 400 may be configured to automatically trigger a validation workflow (e.g., upon start-up of a navigation workflow). The computing device 400 may alternatively or additionally be configured to trigger the validation workflow at predetermined time intervals, e.g., by associating image data with a time stamp and triggering the validation workflow when the age of the youngest time stamp is above a threshold.

Figs. 5A to 5D show a further variant of a length-adjustable and trackable surgical pointer 600, wherein each figure shows the pointer 600 adjusted to a different pointer length. In the following, only the major differences to the previously discussed pointer are explained in detail.

The shaft 650 of the pointer 600 shown in Figs 5A to 5D differs from the rod-like shaft 250 of the pointer 200 shown in Figs. 1 to 3 in that the shaft 650 of the pointer 600 comprises telescopic sections 658, i.e., sections 658 that are movable into and out of each other. Each telescopic section 658 of the pointer 600 comprises a different optical indicator 659. In particular, each section 658 comprises a color optically discernable from the color of the adjacent section(s) 658. Based on the colors (and a respective mapping associated with the colors, as described above for optical patterns), a length of the pointer 600 may be determined as described herein, for example with reference to Fig. 4A.

In the example of Fig. 5, each of the telescopic sections 658 is movable in a translatory manner between two positions, i.e., a section 558 is either moved into or out of the larger neighboring section 658. Thus, the telescopic sections 658 enable identifications of discrete length adjustments, i.e., corresponding to the lengths of the respective telescopic sections 658. In alternatives, one or more of the telescopic sections 658 may comprise a length scale and may be configured for a continuously translational movement, thus allowing identification of a continuous length adjustment. In some variants, the pointer 600 comprises only a single section 658 that can telescopically be moved into and out of tube (see tube 240 of Fig. 2), optionally in combination with length scale.

In other examples (not shown), a pointer shaft 250 may comprise a combination of telescopic sections as shown in Figs 5A to 5D and rod-like sections as shown in Figs 1 to 3B.

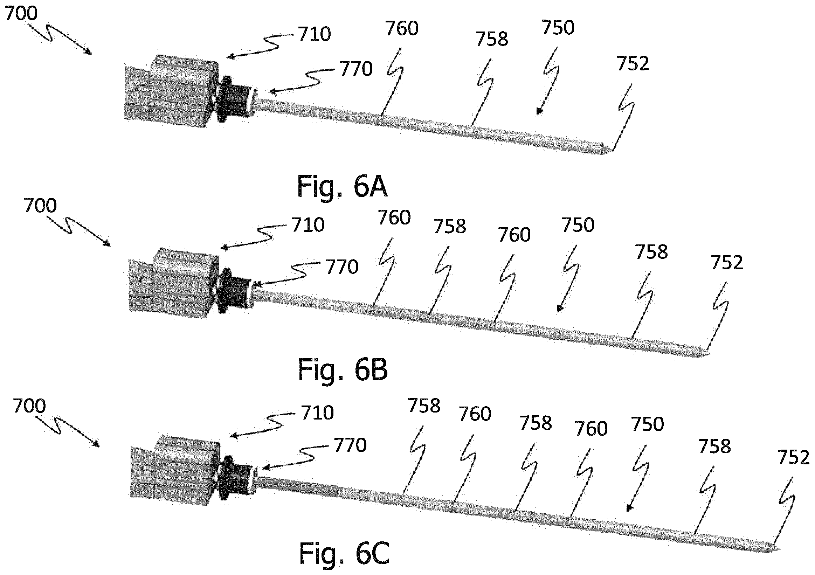

Figs. 6A to 6C show a part of a further length-adjustable surgical pointer 700. Each of Figs. 6A to 6C shows the pointer 700 adjusted to a different length. The pointer 700 comprises a handle 710 and a rod-like shaft 750 similar to the pointer 200 shown in Figs. 1 to 3B. The shaft 750 comprises multiple sections 758 delimited by circumferential depressions 760. The pointer 700 further comprises a locking element 770 configured to be received in (i.e., engage) the depressions 760.

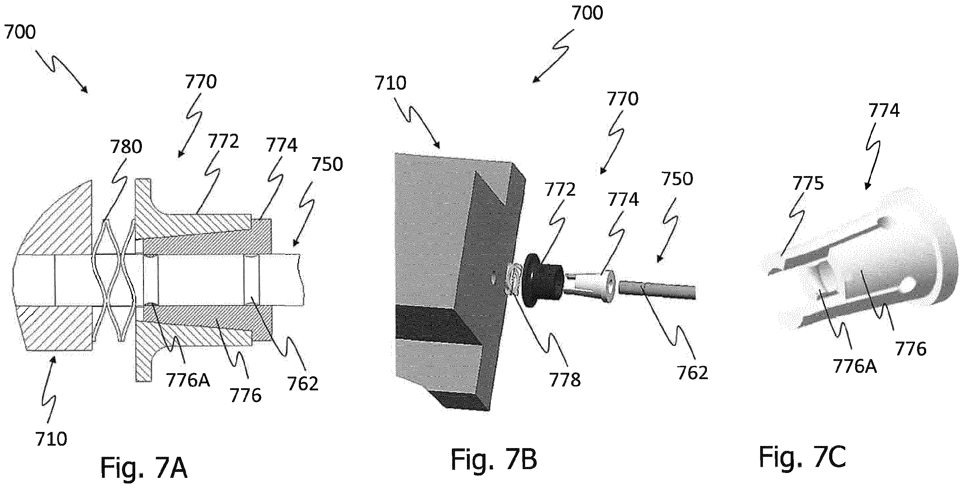

The locking element 770, or parts thereof, is shown in further detail in Figs 7A to 7C. Fig. 7A shows a detailed sectional view of a portion of the pointer 700 of Figs. 6A to 6C comprising the locking element 770. Fig. 7B shows an exploded view of Fig. 7A, and Fig. 7C shows a part of the locking element 770 of Fig. 7A.

The locking element 770 has a substantially hollow-cylindrical form configured to receive the shaft 750 of the pointer 700. The locking element 770 comprises a sleeve portion 772 and a body portion 774. The sleeve portion 772 is configured to be movable over at least a part of a body portion 774. The body portion 774 comprises multiple first arms 775 and multiple second arms 776 distributed circumferentially around the body portion 774, as best shown in Fig. 7C. The second arms 776 are elastically deformable and are configured to move towards each other, i.e., towards a surface of the shaft 750, when the sleeve portion 772 is moved over the body portion. Each second arm 776 comprises a protrusion 776a shaped to be received by the depressions 760 (e.g., in a form-fitting manner).

Further, the sleeve portion 772 is pre-loaded in the distal direction, i.e., away from the handle 710 and towards a pointing tip 752 of the pointer 700, via a spring 780 located between the handle 710 and the sleeve portion 772 so as to move over the body portion 774 when no force is actively biasing the sleeve portion 772 against the spring force of spring 780 towards the handle 710. As a result, the second arms 776 are automatically moved towards the shaft 750 and, in particular, the protrusions 776A are moved inside a depression 760 of the shaft 750 to block translatory movement of the shaft 750 relative to the handle 710. To allow translatory movement of the shaft 750 relative to the handle 710, the sleeve portion 772 has to be moved against the spring force of spring 780 and towards the handle 710, so that the second arms 776 elastically return to their non-deformed state.

As has become apparent from the above description of exemplary embodiments, the length-adjustable pointer presented herein obviates the need for providing a plurality of different fixed-length pointers. Moreover, the length-adjustable pointer can flexibly be adjusted to a desired dimension, e.g., an optimal length, for a particular user and a particular surgical application. The length of the pointer can even be adjusted during an ongoing surgical procedure if the need arises.

The features described in relation to the exemplary embodiments shown in the drawings can be readily combined to result in different embodiments. It is apparent, therefore, that the present disclosure may be varied in many ways. Such variations are not to be regarded as a departure from the scope of the invention as defined by the claims appended hereto.

## Claims

1. A length-adjustable surgical pointer (200, 600, 700) comprising:
a shaft (250, 650, 750) carrying a pointing tip (252, 652, 752);
a handle (210, 610, 710) supporting the shaft (250, 650, 750); and wherein
at least a portion of the shaft (250, 650, 750) is movable in a translatory manner relative to the handle (210, 610, 710) so as to adjust a distance between the pointing tip (252, 652, 752) and the handle (210, 610, 710).

2. The length-adjustable pointer (200, 600, 700) according to claim 1, wherein
the shaft (250, 650, 750) is at least partially movable into or out of the handle (210, 610, 710) for adjusting the distance between the pointing tip (252, 652, 752) and the handle (210, 610, 710).

3. The length-adjustable pointer (200, 600, 700) according to any preceding claim, wherein
the shaft (250, 650, 750) is at least partially configured to perform a telescopic movement for adjusting the distance between the pointing tip (252, 652, 752) and the handle (210, 610, 710).

4. The length-adjustable pointer (200, 600, 700) according to any preceding claim, wherein
the shaft (250, 650, 750) comprises multiple sections (258, 658, 758), wherein each section (258, 658, 758) has a respective predefined length to enable a discrete adjustment of the distance between the pointing tip (252, 652, 752) and the handle (210, 610, 710) based on the respective predefined lengths.

5. The length-adjustable pointer (200, 600, 700) according to any preceding claim, wherein
the shaft (250, 650, 750) comprises at least one optical indicator (259) configured to optically indicate information associated with the currently adjusted length of the pointer (200, 600, 700).

6. The length-adjustable pointer (200, 600, 700) according to claim 5, wherein
the at least one optical indicator (259) comprises at least one of a length scale, a color coding, and a coding by an optical pattern.

7. The length-adjustable pointer (200, 600, 700) according to claims 4 and 6, wherein
neighboring sections (258, 658, 758) comprise at least one of different colors and different optical patterns.

8. The length-adjustable pointer (200, 600, 700) according to any preceding claim, further comprising
a locking element (242, 770) configured to releasably lock the shaft (250, 650, 750) relative to the handle (210, 610, 710) against an unintentional translatory movement therebetween.

9. The length-adjustable pointer (200, 600, 700) according to claim 8, wherein
one of the shaft (250, 650, 750) and the locking element (242, 770) comprises a preloaded snap-in element (254, 776) and the other one comprises a corresponding receiving element (244, 760) configured to releasably receive the snap-in element (254, 776).

10. The length-adjustable pointer (200, 600, 700) according to claim 9, wherein
the shaft (250, 650, 750) comprises at least one depression (760) forming the receiving element; and
the locking element (770) comprises the preloaded snap-in element (776), wherein the snap-in element (776) is configured to engage the at least one depression (760).

11. The length-adjustable pointer (200, 600, 700) according to any of claims 8 to 10, wherein
the locking element (242, 770) is manually actuatable to at least one of lock or unlock the shaft (250, 650, 750) relative to the handle (210, 610, 710).

12. The length-adjustable pointer (200, 600, 700) according to any preceding claim, further comprising
a tracker (290) configured to be detected by a surgical tracking system, or an interface for receiving the tracker (290).

13. A system (100) comprising:
the length-adjustable pointer (200, 600, 700) according to any one of claims 5 to 7 or of claims 8 to 12 when depending on any one of claims 5 to 7; and
a camera (300) configured to generate image data indicative of the at least one optical indicator (259).

14. The system (100) according to claim 13, further comprising:
a computing device (400) configured to receive the image data and determine information associated with a currently adjusted length of the pointer (200, 600, 700) based at least in part on the at least one optical indicator (259) indicated in the received image data.

15. The system of claim 14, wherein
the computing device is configured to determine the information associated with the currently adjusted length of the pointer (200, 600, 700) by
- detecting the at least one optical indicator (259) indicated in the image data; and
- consulting a mapping between (i) different optical indicators (259) and (ii) different items of information associated with adjusted pointer lengths.

16. The system (100) according to any of claims 13 to 15 when depending from at least claim 12, wherein
the computing device (400) is configured to receive data indicative of a pose of the tracker (290) and to generate navigation instructions based at least in part on the information associated with the currently adjusted length of the pointer (200, 600, 700) and the indicated pose.

17. A computer-implemented method for determining information associated with a currently adjusted length of the length-adjustable pointer (200, 600, 700) according to any one of claims 5 to 7 or of claims 8 to 12 when depending on any one of claims 5 to 7, the method (500) comprising:
receiving (510) image data indicative of the at least one optical indicator (259); and
determining (520) the information associated with the currently adjusted length of the pointer (200, 600, 700) based at least in part on the at least one optical indicator (259) indicated in the received image data.

18. The method (500) according to claim 17 when depending from at least claim 12, further comprising:
receiving (510) data indicative of a pose of the tracker (290);
determining (520) navigation instructions based at least in part on the information associated with the currently adjusted length of the pointer (200, 600, 700) and the indicated pose.

19. A computer program product comprising instructions which, when the program is executed by a processor, cause the processor to carry out the method (500) of any of claims 17 to 18.
